# EUROPEAN PATENT SPECIFICATION

(11) **EP 2 470 240 B1**
(45) Date of publication and mention of the grant of the patent: **03.06.2015**
(21) Application number: 10745240.1
(22) Date of filing: 20.08.2010
(51) Int. Cl.: A61M 5/24, A61M 5/34, A61M 5/315, A61M 5/20

(54) **ARRANGEMENT FOR DELIVERING A FLUID MEDICAMENT**
ANORDNUNG FÜR DIE VERABREICHUNG EINES FLÜSSIGEN MEDIKAMENTS
DISPOSITIF D'ADMINISTRATION DE MÉDICAMENT LIQUIDE

(30) Priority: 27.08.2009 EP 09010972
(43) Date of publication of application: 04.07.2012
(73) Proprietor: Sanofi-Aventis Deutschland GmbH, 65929 Frankfurt am Main (DE)
(72) Inventor: NAGEL, Thomas, 01737 Tharandt (DE); RICHTER, René, 01737 Tharandt (DE); WITT, Robert, 01159 Dresden (DE)
(86) International application number: PCT/EP2010/062152
(87) International publication number: WO 2011/023630

(56) References cited:
- WO-A1-92/15345
- WO-A2-98/34657
- US-A- 2 392 196
- US-A- 4 522 622
- US-A1- 2008 058 732
- US-B1- 6 585 693

## Description

The invention relates to an arrangement for delivering a fluid medicament according to the preamble of claim 1.

Many medicaments have to be injected into the body. This applies in particular to medicaments, which are deactivated or have their efficiency remarkably decreased by oral administration, e.g. proteines (such as Insulin, growth hormones, interferons), carbohydrates (e.g. Heparin), antibodies and the majority of vaccines. Such medicaments are predominantly injected by means of syringes, medicament pens or medicament pumps.

In the state of the art medicaments to be injected are stored in medicament containers like cartridges for injecting devices or ampoules.

US 6,585,693 discloses a unitary syringe having a mounted needle surrounded by a sheath having a break-zone. The fluid medicament to be injected is contained within a blister cavity formed between upper and lower polymeric layers. The syringe is part of an array of individual syringes joined together by thin webs.

It is an object of the present invention to provide an improved arrangement for delivering a fluid medicament

The object is achieved by an arrangement according to claim 1.

Preferred embodiments of the invention are given in the dependent claims.

According to the invention, an arrangement for delivering a fluid medicament comprises a dosage apparatus and a medicament container. The medicament container, in particular for an injection device or a dosage apparatus comprises an array of separate flexible reservoirs. Each reservoir comprises a defined charge of a fluid medicament and each reservoir is separately dischargeable. The reservoirs are flexible and connected by a connecting element. The dosage apparatus comprises a releasing actor designed to activate the release of the charge of fluid medicament from a reservoir to a needle. The dosage apparatus comprises a transportation arrangement designed to provide the releasing actor with reservoirs to be released or designed to move the releasing actor to a reservoir to be released next.

In an example embodiment, the releasing actor comprises one or more plungers or uses compressed air. The one or more plungers may correspond to the reservoirs in a one-to-one relationship, so that one plunger corresponds with one reservoir, respectively.

Preferably the connecting element carries six flexible reservoirs; a standard package contains five medicament containers with six flexible reservoirs per medicament container. It is possible to arrange medicament containers with more or less than six flexible reservoirs. A quantum of the fluid medicament for a plurality of injections for a patient is split into separated charges, which are contained in the separated reservoirs. Compared with flexible medicament containers for a quantum of a fluid medicament for a plurality of injections known in the state of the art the claimed medicament container allows to split the quantum of the fluid medicament for a plurality of injections for a patient into a plurality of separated charges in separate flexible reservoirs. Thus, it is possible to discharge a certain number of flexible reservoirs to achieve a defined quantum of the fluid medicament. Preferably the reservoirs are filled with separate charges that are customised to an individual patient. The smallest single charge in one reservoir represents the smallest partial dose of the fluid medicament. That means, according to the quantum of fluid medicament needed a certain number of flexible reservoirs is to be discharged.

In a preferred embodiment of the invention the connecting element of the medicament container is a carrier element which carries the separate reservoirs. According to that embodiment the carrier element as the connecting element is arranged in a manner of a blister pack. Each reservoir contains a defined charge of the medicament. Preferably the reservoirs are arranged at one side of the carrier element as the connecting element. According to an alternative of that embodiment the connecting element is arranged in a manner of a bubble wrap, wherein each reservoir contains a defined charge of the medicament.

The reservoirs of a medicament container according to the invention comprise identical or different charges.

Preferably the array of reservoirs of the medicament container according to the invention is arranged as a band in one or more lines. Thus, it is possible to move the medicament container by translation reservoir by reservoir to discharge a certain number of flexible reservoirs to achieve a defined quantum of the fluid medicament. Alternatively the array of reservoirs of the medicament container according to the invention is arranged as a revolver. Thus, it is possible to move the medicament container by rotation reservoir by reservoir to discharge a certain number of flexible reservoirs to achieve a defined quantum of the fluid medicament. Furthermore it is possible not to move the medicament container by translation or by rotation but to move a plunger reservoir by reservoir to discharge a certain number of flexible reservoirs.

Preferably one or more needles are integrated into the medicament container to increase the efficiency of discharging. According to an alternative a needle is provided for each reservoir. Each needle is arranged inside or outside a corresponding reservoir.

Preferably one or more needles arranged outside a corresponding reservoir are rotatable from a safe inactivated attached first position into an activated second position. The reservoirs of the medicament container can be easily discharged when a corresponding needle is in the second position.

According to the invention preferably each reservoir comprises a predetermined breaking point. That breaking point is designed to release the charge of the fluid medicament to a needle.

According to the invention, the dosage apparatus for discharging at least a part of a medicament container comprises a releasing actor. The dosage apparatus can be used for inhalation devices. Alternatively the dosage apparatus can be part of an injection device. The releasing actor is designed to activate the release of the charge of the fluid medicament from a reservoir to a needle. Preferably the size of the releasing actor is customised according to the largest of the reservoirs. As a releasing actor one or more plungers can be used. A plunger corresponds with a reservoir to be discharged. It is possible to use one plunger to discharge all reservoirs. According to an alternative of the invention compressed air is used as a releasing actor in the dosage apparatus. Preferably the dosage apparatus comprises a releasing actor designed to activate the release of the charge of the fluid medicament from a reservoir. Preferably the fluid medicament is discharged from the reservoir to a needle.

Preferably the dosage apparatus comprises a transportation arrangement designed to provide the one or more plungers with reservoirs to be released. Alternatively the dosage apparatus comprises a transportation arrangement designed to move the plunger to the next reservoir to be released.

An injection device for discharging at least a part of a medicament container comprises the releasing actor. That releasing actor is designed to activate the release of the charge of the fluid medicament from a reservoir to a needle. Preferably the size of the releasing actor is customised according to the largest of the reservoirs. As a releasing actor one or more plungers can be used. A plunger corresponds with a reservoir to be discharged. It is possible to use one plunger to discharge all reservoirs. The injection device comprises one or more needles. The needle can be removable in order to allow a patient to fix a new needle to the injection devise in order to use a new needle for each injection. According to an alternative of the invention compressed air is used as a releasing actor in the injection device.

Preferably the injection device comprises a transportation arrangement designed to provide the one or more plungers with reservoirs to be released. Alternatively the injection device comprises a transportation arrangement designed to move the plunger to the next reservoir to be released.

According to the invention the injection device or the dosage apparatus is used for delivering one of an analgetic, an anticoagulant, an insulin, an insulin derivate, heparin, Lovenox, a vaccine, a growth hormone and a peptide hormone.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

The present invention will become more fully understood from the detailed description given herein below and the accompanying drawings which are given by way of illustration only, and thus, are not limitive of the present invention, and wherein:
- Figure 1a: is a perspective view of a first embodiment of a medicament container,
- Figure 1b: is a cross sectional according to figure 1 a,
- Figure 2a: is a perspective view of a first embodiment of a medicament container,
- Figure 2b: is a cross sectional according to figure 1 a,
- Figure 3: is a schematic view of a third embodiment of a medicament container with a part of an injection device,
- Figure 4: is a schematic view of a forth embodiment of a medicament container with a part of an injection device.

Corresponding parts are marked with the same reference symbols in all figures.

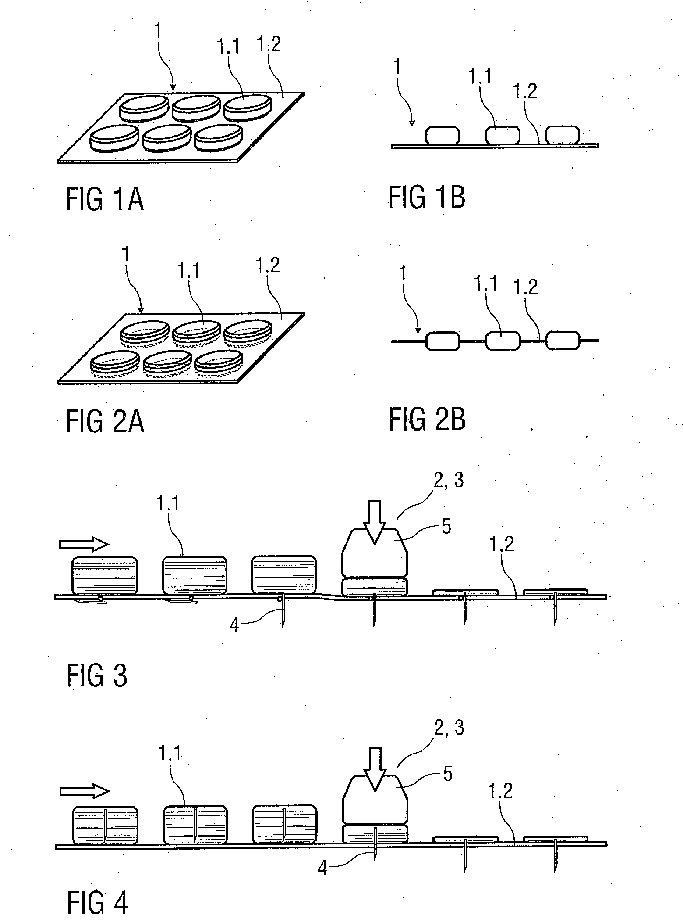

Figure 1 a shows a perspective view of a first embodiment of a medicament container 1 with six reservoirs 1.1 and a connecting element 1.2.

A quantum of a fluid medicament is split into six separate identical charges, which are contained in flexible reservoirs 1.1 made of plastic. These charges are customised to an individual patient. One single charge in a reservoir 1.1 represents the smallest partial dose of the fluid medicament.

The connecting element 1.2 is a carrier element made of plastics. The medicament container 1 with six reservoirs 1.1 and the connecting element 1.2 is arranged in a manner of a blister pack. The reservoirs 1.1 are arranged at one side of the carrying connecting element 1.2.

Figure 1b shows a cross sectional of a medicament container 1 with six reservoirs 1.1 and a connecting element 1.2 according to figure 1 a.

The flexible reservoirs 1.1 filled with fluid medicament are fixed at the upper side of the carrying connecting element 1.2. Each reservoir 1.1 comprises a predetermined breaking point, which is not shown.

Figure 2a shows a perspective view of a second embodiment of the medicament container 1 with six reservoirs 1.1 and the connecting element 1.2.

A quantum of a fluid medicament is split into six separate identical charges, which are contained in flexible reservoirs 1.1 made of thin plastic. These charges are customised to an individual patient. One single charge in a reservoir 1.1 represents the smallest partial dose of the fluid medicament.

The connecting element 1.2 is a flexible element made of thin plastics in a manner of a film. The medicament container 1 with six reservoirs 1.1 and the connecting element 1.2 is arranged in a manner of a bubble wrap.

Figure 2b shows a cross sectional of a medicament container 1 with six reservoirs 1.1 and a connecting element 1.2 according to figure 2a.

The flexible reservoirs 1.1 filled with fluid medicament are connected by the film as a connecting element 1.2.

Figure 3 shows a schematic view of a third embodiment of the medicament container 1 with a part of an injection device 2.

The medicament container 1 consists of six flexible reservoirs 1.1 made of plastic. The six flexible reservoirs 1.1 are arranged in a line and contain the fluid medicament in identical charges. The connecting element 1.2 is a carrier element made as described in Figures 1 a and 1 b. The medicament container 1 is arranged in a manner of a blister pack. The reservoirs 1.1 are arranged at one side of the carrying connecting element 1.2.

At the undersurface of the connecting element 1.2 six needles 4 are integrated into the medicament container 1 whereby one needle 4 is provided for a corresponding reservoir 1.1. Each needle 4 is arranged outside its corresponding reservoir 1.1. The needles 4 are rotatable from a safe inactivated attached first position into a activated second position. The first position of needles 4 is shown at the first and the second reservoir 1.1. Under the third reservoir 1.1 a needle 4 is shown in the second position ready to penetrate the third reservoir 1.1 in order to discharge that third reservoir 1.1. Under the fourth reservoir 1.1 a needle 4 is shown in the second position penetrating the fourth reservoir 1.1 and discharging the fourth reservoir 1.1. Under the fifth and the sixth reservoir 1.1 a needle 4 is shown in the second position after penetration and after discharging the fifth and the sixth reservoir 1.1.

The fourth reservoir 1.1 is being discharged by a plunger 5. The plunger 5 is a releasing actor as a part of an injection device 2 for discharging at least a part of a medicament container 1. The plunger 5 is designed to activate the release of the charge of the fluid medicament from the reservoir 1.1 to the corresponding needle 4. The size of the plunger 5 is customised according to the reservoirs 1.1. The one plunger 5 is used to discharge all reservoirs 1.1. The injection device 2 comprises a transportation arrangement that is not shown. That transportation arrangement is designed to provide the plunger 5 with one reservoir 1.1 after the next to release these reservoirs 1.1.

Alternatively the injection device 2 can comprise a transportation arrangement designed to move the plunger 5 to the next reservoir 1.1 to be released.

The plunger 5 can also be a releasing actor as a part of a dosage apparatus 3 for discharging at least a part of a medicament container 1.

Figure 4 shows a schematic view of a forth embodiment of the medicament container 1 with a part of an injection device 2.

The part of the injection device 2 shown in figure 4 is the same injection device 2 as shown in figure 3.

The medicament container 1 is similar to the medicament container 1 shown in figure 3.

Six needles 4 are integrated into the medicament container 1 whereby one needle 4 is provided for a corresponding reservoir 1.1. Each needle 4 is arranged inside its corresponding reservoir 1.1. The length of each needle 4 is shorter than the height of the corresponding reservoir 1.1 whereby the ends of the needles 4 do not touch the wall of the reservoirs 1.1 when the reservoirs 1.1 are filled. In that first position the needles 4 are inactivated. The first position of needles 4 is shown at the first, the second and the third reservoir 1.1. In the fourth reservoir 1.1 a needle 4 is shown in the second position penetrating the fourth reservoir 1.1 for discharging the fourth reservoir 1.1. Under the fifth and the sixth reservoir 1.1 a needle 4 is shown in the second position after penetration and after discharging the fifth and the sixth reservoir 1.1.

The fourth reservoir 1.1 is being discharged by a plunger 5. The plunger 5 is a releasing actor as a part of an injection device 2 for discharging at least a part of a medicament container 1 as described in figure 3.

Alternatively the injection device 2 can comprise a transportation arrangement designed to move the plunger 5 to the next reservoir 1.1 to be released.

The plunger 5 can also be a releasing actor as a part of a dosage apparatus 3 for discharging at least a part of a medicament container 1.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28)-human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω)-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(0)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(0)14 Trp(02)25, Asp28] Exendin-4(1-39),
   des Pro36 [Met(0)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2;
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(0)14, Asp28] Exendin-4(1-39)-Lys6-NH2,
des Met(0)14 Asp28 Pro36, Pro37, Pro38 Exendin-4(1-39)-NH2,
H-(Lys)6-desPro36, Pro37, Pro38 [Met(0)14, Asp28] Exendin-4(1-39)-NH2,
H-Asn-(Glu)5-des Pro36, Pro37, Pro38 [Met(0)14, Asp28] Exendin-4(1-39)-NH2,
des Pro36, Pro37, Pro38 [Met(0)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2;
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(0)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(0)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

### List of References

- 1: medicament container
- 1.1: reservoir
- 1.2: connecting element
- 2: injection device
- 3: dosage apparatus
- 4: needle
- 5: plunger

## Claims

1. Arrangement for delivering a fluid medicament, the arrangement comprising a medicament container (1), the medicament container (1) comprising an array of separated reservoirs (1.1) wherein each reservoir (1.1) comprises a defined charge of a fluid medicament, wherein each reservoir (1.1) is separately dischargeable and wherein the reservoirs (1.1) are flexible and connected by a connecting element (1.2), **characterized in that** the arrangement furthermore comprises a dosage apparatus (3) comprising a releasing actor designed to activate the release of the charge of fluid medicament from a reservoir (1.1) to a needle (4), wherein the dosage apparatus comprises a transportation arrangement designed to provide the releasing actor (5) with reservoirs (1.1) to be released or designed to move the releasing actor (5) to a reservoir (1.1) to be released next.

2. Arrangement according to claim 1, **characterized in that** the releasing actor (5) comprises one or more plungers or uses compressed air.

3. Arrangement according to claims 2, wherein one plunger (5) corresponds with one reservoir (1.1), respectively.

4. Arrangement according to one of the preceding claims, **characterized in that** an injection device (2) comprises the dosage apparatus (3) and at least one needle.

5. Arrangement according to one of the preceding claims, **characterized in that** the connecting element (1.2) is a carrier element which carries the separated reservoirs (1.1) and is arranged in a manner of a blister pack.

6. Arrangement according to one of the preceding claims, **characterized in that** the reservoirs (1.1) comprise identical charges.

7. Arrangement according to one of the claims 1 to 5, **characterized in that** the reservoirs (1.1) comprise different charges.

8. Arrangement according to one of the preceding claims, **characterized in that** the array of reservoirs (1.1) is arranged as a band in one or more lines or as a revolver.

9. Arrangement according to one of the preceding claims, **characterized in** one or more integrated needles (4).

10. Arrangement according to claim 9, **characterized in that** a needle (4) is provided for each reservoir (1.1) whereby each needle (4) is arranged inside or outside a corresponding reservoir (1.1).

11. Arrangement according to claim 9 or 10, **characterized in that** the one or more needles (4) are arranged outside a corresponding reservoir (1.1) and are rotatable from an inactivated attached first position into an activated second position.

12. Arrangement according to one of the preceding claims, **characterized in that** each reservoir (1.1) comprises a predetermined breaking point designed to release the charge of the fluid medicament to a needle (4).

## Patentansprüche

1. Anordnung zum Verabreichen eines fluiden Medikaments, wobei die Anordnung einen Medikamentbehälter (1) umfasst, wobei der Medikamentbehälter (1) ein Array von getrennten Reservoirs (1.1) umfasst, wobei jedes Reservoir (1.1) eine definierte Füllung eines fluiden Medikaments umfasst, wobei jedes Reservoir (1.1) getrennt entleerbar ist und wobei die Reservoirs (1.1) flexibel und über ein Verbindungselement (1.2) verbunden sind, **dadurch gekennzeichnet, dass** die Anordnung ferner eine Dosiervorrichtung (3) umfasst, die einen Freigabebetätiger umfasst, der dazu ausgelegt ist, das Freigeben der Füllung des fluiden Medikaments aus einem Reservoir (1.1) an eine Nadel (4) zu aktivieren, wobei die Dosiervorrichtung eine Transportanordnung umfasst, die dazu ausgelegt ist, den Freigabebetätiger (5) mit freizugebenden Reservoirs (1.1) zu versorgen, oder dazu ausgelegt ist, den Freigabebetätiger (5) zu einem Reservoir (1.1) zu bewegen, das als nächstes freizugeben ist.

2. Anordnung nach Anspruch 1, **dadurch gekennzeichnet, dass** der Freigabebetätiger (5) einen oder mehrere Kolben umfasst oder Druckluft einsetzt.

3. Anordnung nach Anspruch 2, wobei ein Kolben (5) jeweils einem Reservoir (1.1) entspricht.

4. Anordnung nach einem der vorhergehenden Ansprüche, **dadurch gekennzeichnet, dass** eine Injektionsvorrichtung (2) die Dosiervorrichtung (3) und mindestens eine Nadel umfasst.

5. Anordnung nach einem der vorhergehenden Ansprüche, **dadurch gekennzeichnet, dass** das Verbindungselement (1.2) ein Trägerelement ist, das die getrennten Reservoirs (1.1) trägt und in der Art einer Blisterpackung angeordnet ist.

6. Anordnung nach einem der vorhergehenden Ansprüche, **dadurch gekennzeichnet, dass** die Reservoirs (1.1) identische Füllungen umfassen.

7. Anordnung nach einem der Ansprüche 1 bis 5, **dadurch gekennzeichnet, dass** die Reservoirs (1.1) unterschiedliche Füllungen umfassen.

8. Anordnung nach einem der vorhergehenden Ansprüche, **dadurch gekennzeichnet, dass** das Array von Reservoirs (1.1) als ein Band in einer oder mehreren Linien oder als Revolver angeordnet ist.

9. Anordnung nach einem der vorhergehenden Ansprüche, **gekennzeichnet durch** eine oder mehrere integrierte Nadeln (4).

10. Anordnung nach Anspruch 9, **dadurch gekennzeichnet, dass** eine Nadel (4) für jedes Reservoir (1.1) vorgesehen ist, wobei jede Nadel (4) innerhalb oder außerhalb eines entsprechenden Reservoirs (1.1) angeordnet ist.

11. Anordnung nach Anspruch 9 oder 10, **dadurch gekennzeichnet, dass** die eine oder die mehreren Nadeln (4) außerhalb eines entsprechenden Reservoirs (1.1) angeordnet und aus einer deaktivierten angebrachten ersten Position in eine aktivierte zweite Position drehbar sind.

12. Anordnung nach einem der vorhergehenden Ansprüche, **dadurch gekennzeichnet, dass** jedes Reservoir (1.1) eine vorbestimmte Bruchstelle umfasst, die dazu ausgelegt ist, die Füllung des fluiden Medikaments an eine Nadel (4) freizugeben.

## Revendications

1. Agencement pour administrer un médicament fluide, l'agencement comprenant un récipient de médicament (1), le récipient de médicament (1) comprenant un ensemble de réservoirs séparés (1.1), chaque réservoir (1.1) comprenant une charge définie d'un médicament fluide, chaque réservoir (1.1) pouvant être déchargé séparément et les réservoirs (1.1) étant flexibles et connectés par un élément de connexion (1.2), **caractérisé en ce que** l'agencement comprend en outre un appareil de dosage (3) comprenant un actionneur de libération conçu pour activer la libération de la charge de médicament fluide d'un réservoir (1.1) à une aiguille (4), l'appareil de dosage comprenant un agencement de transport conçu pour fournir à l'actionneur de libération (5) des réservoirs (1.1) destinés à être libérés ou conçus pour déplacer l'actionneur de libération (5) jusqu'à un réservoir (1.1) devant ensuite être libéré.

2. Agencement selon la revendication 1, **caractérisé en ce que** l'actionneur de libération (5) comprend un ou plusieurs plongeurs ou utilise de l'air comprimé.

3. Agencement selon la revendication 2, dans lequel un plongeur (5) correspond à un réservoir (1.1), respectivement.

4. Agencement selon l'une quelconque des revendications précédentes, **caractérisé en ce qu'**un dispositif d'injection (2) comprend l'appareil de dosage (3) et au moins une aiguille.

5. Agencement selon l'une quelconque des revendications précédentes, **caractérisé en ce que** l'élément de connexion (1.2) est un élément de support qui porte les réservoirs séparés (1.1) et qui est agencé à la manière d'une plaquette thermoformée.

6. Agencement selon l'une quelconque des revendications précédentes, **caractérisé en ce que** les réservoirs (1.1) comprennent des charges identiques.

7. Agencement selon l'une quelconque des revendications 1 à 5, **caractérisé en ce que** les réservoirs (1.1) comprennent différentes charges.

8. Agencement selon l'une quelconque des revendications précédentes, **caractérisé en ce que** l'ensemble de réservoirs (1.1) est agencé sous forme de bande en une ou plusieurs lignes ou sous forme de tourelle.

9. Agencement selon l'une quelconque des revendications précédentes, **caractérisé par** une ou plusieurs aiguilles intégrées (4).

10. Agencement selon la revendication 9, **caractérisé en ce qu'**une aiguille (4) est prévue pour chaque réservoir (1.1), chaque aiguille (4) étant agencée à l'intérieur ou à l'extérieur d'un réservoir correspondant (1.1).

11. Agencement selon la revendication 9 ou 10, **caractérisé en ce que** la ou les aiguilles (4) sont agencées à l'extérieur d'un réservoir correspondant (1.1) et peuvent tourner d'une première position attachée non activée dans une deuxième position activée.

12. Agencement selon l'une quelconque des revendications précédentes, **caractérisé en ce que** chaque réservoir (1.1) comprend un point de rupture prédéterminée conçu pour libérer la charge du médicament fluide vers une aiguille (4).
